Europäisches Patentamt

European Patent Office

Office européen des brevets

(19)

(11) Publication number: **0 254 647**
**A2**

# (12) EUROPEAN PATENT APPLICATION

(21) Application number: **87401724.7**

(22) Date of filing: **23.07.87**

(51) Int. Cl.4: **A 61 K 37/02**

(30) Priority: **25.07.86 JP 175156/86**

(43) Date of publication of application:
**27.01.88 Bulletin 88/04**

(84) Designated Contracting States: **DE FR GB IT**

(71) Applicant: **MITSUBISHI CHEMICAL INDUSTRIES LIMITED**
**5-2, Marunouchi 2-chome Chiyoda-ku Tokyo 100 (JP)**

(72) Inventor: **Sugamura, Kazuo**
**3-1-15 Kashiwagi**
**Sendai-shi Miyagi-ken (JP)**

**Muramatsu, Minoru**
**1-18-14 Fujigaoka Midori-ku**
**Yokohama-shi Kanagawa-ken (JP)**

**Honda, Kumiko**
**3-4-13 Shimoishihara**
**Chofu-shi Tokyo (JP)**

**Nakagawa, Yoshio**
**2-15-6 Higashi-Tamagawagakuen**
**Machida-shi Tokyo (JP)**

(74) Representative: **Gutmann, Ernest et al**
**S.C. Ernest Gutmann - Yves Plasseraud 67, boulevard Haussmann**
**F-75008 Paris (FR)**

(54) Medicines for treatment of autoimmune diseases.

(57) The present invention relates to medicines for the treatment of autoimmune diseases, more particularly systemic lupus erythematosus (SLE). The medicine according to the invention comprises a cytotoxic cytokine, such as tumor necrosis factor (TNF) or lymphotoxin, as an effective ingredient.

EP 0 254 647 A2

**Description**

MEDICINES FOR TREATMENT OF AUTOIMMUNE DISEASES

BACKGROUND OF THE INVENTION

Field of the Invention:

The present invention relates to a medicine for the treatment of autoimmune diseases.

Description of the Prior Art:

Autoimmune diseases may be considered to be phenomena in which the immunological tolerance (non-reactivity) to autoantigens is destroyed by any factor capable of inducing or causing the self response to the autoantigens.

Such inducing factors that have already been known may include an encounter of the immune system with a sequestered autoantigen, the appearance of a forbidden clone, the T cell bypass through a modified autoantigen or a cross-reactive antigen, activation of a non-specific lymphocyte, inadequacy of suppressor T cells, and a disorder of the anti-idiotype network.

One of notable symptoms which may be observed in autoimmune diseases is self-toxicity. Such a symptom, self-toxicity (cytotoxicity), may be classified into the complement-dependent cytotoxic activity, the phagocyte-dependent cytotoxic activity, the killer cell-dependent cytotoxic activity, the cytotoxic T cell-dependent cytotoxic activity, or the like. However, detailed analysis of the symptom has not been completed.

It may also be considered that immune complexes should be involved in autoimmune diseases: for instance, a DNA-anti-DNA antibody complex may be involved in nephropathy or angiitis caused by systemic lupus erythematosus (SLE); and, an immune complex containing a rheumatoid factor may be involved in rheumatoid arthritis.

It has been known that such autoimmune diseases are anyway controlled genetically; however, details have not been analyzed yet.

As stated above, the mechanism of development of an autoimmune disease is complicated and details thereof have scarcely been found now. Thus, no effective method for treating such an autoimmune disease has been discovered.

On the other hand, cytotoxic cytokines which are considered to be effective in treating cancer include monocyte-derived substances represented by tumor necrosis factor (TNF) and T- or B-cell derived substances represented by lymphotoxin.

These cytotoxic cytokines have been considered to have no activity of so-called biological response modifier (BRM) for acting on immune systems to modify their response, but they may act directly on cancer cells to necrose selectively the cancer cells: Nippon Gan Gakkai (Japan Cancer Association), the 43rd general meeting, p. 273, speech No. 965 (1984).

SUMMARY OF THE INVENTION

The present inventors have now discovered that cytotoxic cytokines are effective in the treatment of autoimmune diseases, particularly systemic lupus erythematosus (SLE), as immune regulating factors. Thus, the present invention has been attained.

According to the invention, there are provided medicines for the treatment of autoimmune diseases, which contain cytotoxic cytokines as effective ingredients.

DESCRIPTION OF THE INVENTION

The invention will hereinbelow be described in detail.

The cytotoxic cytokines used in the invention are proteins having in vitro direct cytotoxic activity against mouse L-cell line and/or in vivo anti-tumor activity against Meth A sarcoma transplanted tumor mouse.

Carswell et al. found that in mice sensitized with Bacillus Calmette Guerin (BCG), when endotoxin was administered on the 14th day after sensitization, an agent having a cytotoxic activity to L-cell was produced in the sera two hours after the administration, and they named the agent tumor necrosis factor (TNF): Proc. Nat. Acad. Sci., USA, 72, 3666 (1975).

Since Carswell et al. discovery, various substances exhibiting the cytotoxicity against L-cell have been reported. Among them, TNF which is a representative of the monocyte-derived cytotoxic cytokines and lymphotoxin which is a representative of the T-cell or B-cell-derived cytotoxic cytokines have been isolated. Aggarwal et al. reported proteinous properties of the cytokines: J. Biol. Chem., 260, 2345, (1985); and J. Biol. Chem., 259, 686 (1984).

The cytotoxic cytokines used in the present invention may include various unidentified substances as well as the aforementioned isolatable TNF and lymphotoxin. Thus, leukoregulins described by Janet H. Ransom et al. in Cancer Research, 45, 851 (1985); human-derived cytotoxic agents described by Hava Neumann in Biochemical Journal, 194, 847 (1981); human endogenous cancer regulating agents disclosed in Japanese Patent Application Laying-open (KOKAI) No. 19720/85; and the like are also contemplated in the invention.

Autoimmune diseases referred to in the present invention may be defined as immune complex diseases. The immune complex diseases are those caused by immune complexes which result from the specific reaction of antigens with antibodies and will do damages to tissues. Such diseases may include systemic lupus erythematosus (SLE), rheumatoid arthritis, thyroiditis and the like.

These cytotoxic cytokines as medicines for the treatment of autoimmune diseases may be administered via any known route except oral, preferably intravenously or intraperitoneally.

Dosage form may be injection prepared by dissolving the cytotoxic cytokine in a solution having a composition suitable for known injectable solutions. The concentration of such an injection is usually 0.1 to 10 mg/ml.

Dose amount may generally be 10,000 to 10,000,000 units per dose, preferably 100,000 to 1,000,000 units per dose. The unit may be determined by the cytotoxicity test using L-cell (J. Biol. Chem., 259, 686 (1984)). The number of doses per day may suitably be determined according to the symptom.

## EXAMPLES

The invention will be further illustrated by the following examples. These examples are not intended to limit the scope of the invention defined in the appended claims.

## REFERENCE EXAMPLE: Preparation of cytotoxic cytokines

TNF was prepared from the supernatant of a culture of human monocyte precursor cell line HL-60 according to the method described by Aggarwal et al. in J. Biol. Chem., 260, 2345 (1985). Lymphotoxin was prepared from the supernatant of a culture of human B cell transformed with EB virus according to the method described by Aggarwal et al. in J. Biol. Chem., 259, 686 (1984).

SDS electrophoresis and reverse phase HPLC (high performance liquid chromatogaphy) showed that the thus prepared cytotoxic cytokines contained substantially no other cytokine.

## EXAMPLE: Treatment of SLE mice with cytotoxic cytokines

Effect of the cytotoxic cytokines on autoimmune diseases was estimated by improvement in one or more symptoms resulting from the autoimmune diseases which had already been reported on one strain of SLE mice (MRL/lpr mice). More specifically, apothanasia effect, reduction of the level of autoantibodies, elimination of lymphadenia, elimination of splenomegaly, and the like were observed in vivo to estimate the effect.

The animals used in the example were female MRL/lpr mice of 12 week old. To the animals treated, a solution of the cytotoxic cytokine obtained in Reference Example above, TNF or lymphotoxin, in saline (100,000 units per ml) was intravenously administered in an amount of 0.1 ml (10,000 units) per dose every other day. In control group, saline free of cytotoxic cytokine was administered in a like manner. The administration was continued for three weeks and estimation was done in the 15th week. The results are shown in Table below.

3

Table 1:   Treatment of SLE mice with cytotoxic cytokines

| Item | Control | Lymphotoxin | TNF |
| --- | --- | --- | --- |
| Apothanasia effect | 0/3 | 3/3 | 3/3 |
| (survival at 17th week) (all died in 16 weeks) | | | |
| Improvement in | | | |
| Lymphadenia | 0/3 | 2/3 | 1/3 |
| Splenomegaly | 0/3 | 3/3 | 2/3 |
| Supervening obesity | 0/3 | 1/3 | 1/3 |
| Supervening dermatosis | 0/3 | 3/3 | 3/3 |
| Supervening arthritis | 0/3 | 2/3. | 2/3 |
| Supervening nephritis | 0/3 | 3/3 | 2/3 |

Note:   B/A   B=total number of mice showing an improvement;
A=total number of mice used.

As shown in Table above, the medicines according to the present invention are effective in the treatment of autoimmune diseases.

**Claims**

1. A medicine for treating an autoimmune disease, which comprises a cytotoxic cytokine as an effective ingredient.

2. The medicine in accordance with claim 1, in which the cytotoxic cytokine is tumor necrosis factor (TNF).

3. The medicine in accordance with claim 1, in which the cytotoxic cytokine is lymphotoxin.

4. The medicine in accordance with claim 1, in which the autoimmune disease is systemic lupus erythematosus (SLE).

5. A pharmaceutical composition which contains the cytotoxic cytokine of any of claims 1 to 3.

6. The use of a cytotoxic cytokine according to any of claims 1 to 3 in a process for the production of a pharmaceutical composition effective in the treatment of autoimmune disease, particularly of systemic lupus erythematosus (SLE).